**Europäisches Patentamt**

**European Patent Office**

**Office européen des brevets**

(11) Veröffentlichungsnummer: **0 073 055**
**B1**

(12) **EUROPÄISCHE PATENTSCHRIFT**

(45) Veröffentlichungstag der Patentschrift:
06.06.84

(51) Int. Cl.³: **C 07 C  21/12**, C 07 C  17/26

(21) Anmeldenummer: **82107711.2**

(22) Anmeldetag: **23.08.82**

(54) **Verfahren zur katalytischen Umwandlung von Tetrachlorkohlenstoff in Perchlorethylen.**

(30) Priorität: **25.08.81  DE 3133524**

(43) Veröffentlichungstag der Anmeldung:
**02.03.83 Patentblatt 83/9**

(45) Bekanntmachung des Hinweises auf die Patenterteilung:
**06.06.84 Patentblatt 84/23**

(84) Benannte Vertragsstaaten:
**AT DE FR GB IT NL SE**

(56) Entgegenhaltungen:
**US - A - 4 002 695**

**CHEMICAL ABSTRACTS, Band 83, Nr. 21, 24. November 1975, Seite 502, Nr. 178187t, Columbus, Ohio, USA M.I. DYUSENOV et al.: "Preparation of perchloroethylene by carbon tetrachloride pyrolysis"**
**CHEMICAL ABSTRACTS, Band 59, Nr. 7, 30. September 1963, Spalte 7359h, Columbus, Ohio, USA H. STEGNER: "Preparation of tetrachloroethylene from carbon tetrachloride"**
**CHEMICAL ABSTRACTS, Band 92, Nr. 23, 9. Juni 1980, Seite 638, Nr. 197912c, Columbus, Ohio, USA**

(73) Patentinhaber: **WACKER-CHEMIE GMBH, Prinzregentenstrasse 22, D-8000 München 22 (DE)**

(72) Erfinder: **Blum, Klaus, Dr. Dipl.-Chem., Hermann-Hiller-Strasse 47, D-8263 Burghausen (DE)**
Erfinder: **Müller, Erich, Marktler Strasse 25, D-8263 Burghausen (DE)**
Erfinder: **Strasser, Rudolf, Dr. Dipl.-Chem., Lindacher Strasse 58, D-8263 Burghausen (DE)**

0 073 055

**Beschreibung**

Die Erfindung betrifft die katalytische Umwandlung von Tetrachlorkohlenstoff in Perchlorethylen in Gegenwart von Chlorakzeptoren in der Gasphase.

Aus CA, Vol. 59, 7359 h, ist bereits bekannt, daß $CCl_4$ in Gegenwart eines $SiO_2$-Katalysators bei Temperaturen von 600 bis 900°C teilweise in Perchlorethylen umgewandelt wird, wobei jedoch beträchtliche Mengen an Hexachlorethan gebildet werden. In Gegenwart von beispielsweise Wasserstoff wird, insbesondere bei geringem Durchsatz von $CCl_4$, die Ausbeute an Perchlorethylen verbessert. Dabei muß jedoch die vermehrte Bildung von Teerprodukten in Kauf genommen werden, was zu Verstopfungen im Reaktionssystem führt.

Es wurde nun gefunden, daß die Umwandlung von $CCl_4$ in $C_2Cl_4$ in der Gasphase in Gegenwart von Chlorakzeptoren bei Temperaturen zwischen 350 und 550°C an oberflächenreichen Festkörpern, die einen $SiO_2$-Gehalt von 60 bis 100 Gew.-% aufweisen, mit hoher Selektivität in bezug auf das Zielprodukt Perchlorethylen abläuft.

Gegenstand der Erfindung ist ein Verfahren zur Umwandlung von Tetrachlorkohlenstoff in Perchlorethylen, das dadurch gekennzeichnet ist, daß die Umwandlung an oberflächenreichen Festkörpern mit einem analytischen $SiO_2$-Gehalt von 60 bis 100 Gew.-% bei Temperaturen von 350 bis 550°C durchgeführt wird.

Vorzugsweise beträgt die Temperatur 400 bis 500°C.

Unter der anspruchsgemäßen Bezeichnung »oberflächenreiche Festkörper« werden vorzugsweise solche mit einer spezifischen Oberfläche von 100 bis 700 $m^2/g$, insbesondere 200 bis 500 $m^2/g$, verstanden.

Der analytische $SiO_2$-Gehalt der Festkörper beträgt 60 bis 100 Gew.-%.

Im Hinblick auf möglichst hohe Standzeiten der Katalysatoren sind innerhalb der oben charakterisierten Auswahl von Festkörpern chlorierungsstabile Sorten bevorzugt. Es werden daher insbesondere solche, einen analytischen $SiO_2$-Gehalt von 60 bis 100 Gew.% aufweisende oberflächenreiche Festkörper eingesetzt, die zumindest an der Oberfläche einen geringen Gehalt an flüchtige Chloride bildenden Bestandteilen, wie beispielsweise Eisen oder Aluminium, enthalten. Die erfindungsgemäß einzusetzenden Festkörper werden daher ggf. einer solche oberflächenständigen Bestandteile entfernenden Behandlung, wie beispielsweise Calcinieren oder Anlösen, unterzogen. Als weitere Oberflächenbehandlung, die sich auch einem Calcinierungsprozeß anschließen kann, wird oftmals eine Imprägnierung der erfindungsgemäßen Feskörper mit Metallsalzlösung vorgenommen. Es können die Salze der Metalle der 1. und 2. Hauptgruppe und insbesondere Salze der Nebengruppenmetalle, wie beispielsweise Salze des Kupfers, des Mangans, des Chroms, des Molybdäns, des Kobalts, des Nickels, des Rutheniums, des Rhodiums und des Palladiums eingesetzt werden. Die genannten Metallsalze werden durch Adsorption oder Ionenaustausch an den Festkörper gebunden bzw. in den Festkörper eingebaut.

Beispiele für erfindungsgemäße Festkörper, die ggf. einer obenbeschriebenen Behandlung unterworfen wurden, sind gefällte oder pyrogen erzeugte Kieselsäuren, Calcium- und Magnesiumsilikate sowie Alumosilikate — insbesondere aluminiumarme Alumosilikate. Spezielle Beispiele sind Montmorillonit, Bentonit, Beidellit, Kaolin, Halosit und Alumosilikate mit 2- oder 3dimensionaler Porenstruktur, insbesondere Zeolith A, X, Y, D, Mordenit, Erionit.

Die erfindungsgemäßen Katalysatoren werden bevorzugt in einer Festbettanordnung eingesetzt. Hierfür wird die Katalysatorsubstanz zweckmäßigerweise in Formen gepreßt, wie beispielsweise Kugeln, Zylinder, Ringe, Kegel, Würfel und dergleichen.

Beispiele für Chlorakzeptoren, die einzeln oder im Gemisch eingesetzt werden können, sind Wasserstoff, sind Alkane, wie Methan, Ethan, Propan, die verzweigten und unverzweigten Butane und Pentane; Alkene, wie Ethylen, Propen, die verzweigten und unverzweigten Butene und Pentene, Alkine, wie Acetylen, Propin, die verzweigten und unverzweigten Butene und Pentene; partiell chlorierte Kohlenwasserstoffe, wie Chloroform, Ethylchlorid, 1,2-Dichlorethan, Vinylchlorid, Allylchlorid, Dichlorpropan, insbesondere Wasserstoff.

Das Konzentrationsverhältnis von umzuwandelndem Tetrachlorkohlenstoff zu Chlorakzeptor beträgt, ausgedrückt als Molverhältnis, vorteilhafterweise zwischen 1 : 1 und 4 : 1. Dabei wird zweckmäßigerweise im höheren anspruchsgemäßen Temperaturbereich eine relativ hohe Chlorakzeptorkonzentration und im anspruchsgemäßen tieferen Temperaturbereich eine niedrigere Chlorakzeptorkonzentration eingehalten.

Die günstigsten Raumströmungsgeschwindigkeiten liegen im Bereich von 50 bis 1000 g umzuwandelndem Tetrachlorkohlenstoff pro Stunde und pro 1-Liter-Katalysatorvolumen.

Das erfindungsgemäße Verfahren wird im Druckbereich zwischen 0,1 und 10 bar durchgeführt. Vorzugsweise liegt der Druck im Bereich der umgebenden Atmosphäre, also bei etwa 1 bar abs.

Die Durchführung des erfindungsgemäßen Verfahrens erfolgt beispielsweise in einem beheizbaren Rohrreaktor, in den der erfindungsgemäße Katalysator eingebettet ist. Der Rohrrekator besteht aus einer Verdampfer- und Vorwärmzone, in der Tetrachlorkohlenstoff eindosiert und auf die Reaktionstemperatur von 350 bis 550°C gebracht wird, der eigentlichen Reaktionszone und einer nachfolgenden Abkühlzone, die zweckmäßigerweise eine Kühlvorrichtung aufweist, um das Reaktionsprodukt auf

2

eine Temperatur um 220°C abzuschrecken. Wenn auch das Abschrecken des Reaktionsgemisches nicht grundsätzlich erforderlich ist, so ist es doch bei Betrieb im höheren anspruchsgemäßen Temperaturbereich angezeigt, da unter Umständen geringe Mengen an Chlor gebildet werden, die ohne die Maßnahme des Abschreckens zu unerwünschter Nebenproduktbildung führen können.

Chlorakzeptor und Tetrachlorkohlenstoff können zusammen oder getrennt in den Reaktor eingespeist werden. Grundsätzlich können die Reaktanten ($CCl_4$ und Chlorakzeptor) in gasförmigem oder flüssigem Zustand dem Reaktionssystem zugeführt werden. Weiterhin können den Reaktanten geringe Mengen Sauerstoffs beigemischt sein, um Ablagerungen am Katalysator zu verhindern. Die Sauerstoffzugabe kann dabei sowohl kontinuierlich als auch diskontinuierlich erfolgen.

Ferner ist es zur Einstellung optimaler Verweilzeiten und Raumströmungsgeschwindigkeiten oftmals zweckmäßig, dem Reaktionsgemisch ein inertes Trägergas, wie Stickstoff, Kohlendioxid, Argon und dergleichen, zuzumischen.

Frisch eingesetzter Katalysator wird zweckmäßigerweise einer wasserentfernenden Vorbehandlung unterzogen. Dies kann Maßnahmen umfassen, wie Vortrocknen des Produkts bei 150°C und Überleiten eines trockenen Gases bei Reaktionstemperatur. Spuren von Wasser können ferner durch Überleiten von Tetrachlorkohlenstoff bei etwa 300°C entfernt werden.

Ein in Betrieb befindlicher Katalysator wird günstigerweise von Zeit zu Zeit einer regenerierenden Behandlung unterworfen, wie Überleiten eines sauerstoffhaltigen Gases bei 350 bis 800°C oder Waschen des Katalysators mit Tetrachlorkohlenstoff bei Temperaturen von 20 bis 76°C.

Nach dem erfindungsgemäßen Verfahren gelingt es, Tetrachlorkohlenstoff mit hoher Selektivität in Perchlorethylen, bei gegenüber pyrolytischen Prozessen relativ niedrigen Temperaturen umzuwandeln.

Die Erfindung wird nun anhand von Beispielen näher erläutert:

## Beispiel 1

In einen Rohrreaktor aus Nickel (Durchmesser 25 mm, beheizte Rohrlänge 1 m) wurden 500 ml eines Kieselsäurepräparates ($SiO_2$-Gehalt 99,8 Gew.-%, spezifische Oberfläche 300 $m^2/g$,) in Kugelform (Durchmesser 2 mm) eingefüllt. Längs des Rohrreaktors wurde ein Temperaturprofil eingestellt, derart, daß in der eigentlichen, ca. 30 cm langen, Reaktionszone in etwa der Mitte des Rohres (entsprechend ca. 100 ml Katalysatorvolumen) eine Temperatur von 450°C eingehalten wurde und die Temperatur im Innern des Rohres bis zu den Rohrenden auf ca. 250°C abfiel.

In diese Anordnung wurden 40 g/Stunde flüssigen Tetrachlorkohlenstoffs eindosiert zusammen mit 2 l/Stunde Wasserstoff und 2 l/Stunde Stickstoff.

Bei einer Betriebsdauer von 700 Stunden fielen neben 5,6 g/Stunde HCl nach Kondensation des Reaktionsgemisches 32 bis 34 g/Stunde eines Flüssigprodukts an mit der folgenden durch Gaschromatographie ermittelten Produktverteilung:

$CHCl_3$ 0,6 Gew.-%, $CCl_4$ 85,2 Gew.-%, $C_2Cl_4$ 13,6 Gew.-%, $C_2Cl_6$ Spuren, $C_4Cl_6$ 0,02 Gew.-%.

Demnach errechnet sich ein Umsatz von $CCl_4$ zu $C_2Cl_4$ von 21,4 Mol-%, entsprechend einer Selektivität von 82 Mol-%.

## Beispiel 2

Es wurde die Arbeitsweise gemäß Beispiel 1 wiederholt, mit den folgenden Abänderungen:

160 g/Stunde Tetrachlorkohlenstoff, 8 l/Stunde Wasserstoff und 1 l/Stunde Luft wurden bei 500°C über den Kontakt geleitet. Neben 22,5 g/Stunde HCl fielen 133,2 g Kondensat folgender Zusammensetzung an:

$CCl_4$ 75,7 Gew.-%, $C_2Cl_4$ 12,9 Gew.-%, $C_2Cl_6$ 9,2 Gew.-%, $C_4Cl_6$ 0,6 Gew.-%, $C_6Cl_6$ 0,3 Gew.-%.

Demnach beträgt der Umsatz von $CCl_4$ zu $C_2Cl_4$ 20,1 Mol-% mit einer Selektivität von 62 Mol-%.

## Beispiel 3

227 g des gemäß Beispiel 1 beschriebenen Kieselsäureprodukts wurden in einer Lösung von 50 g KCl in 1 l Wasser 12 Stunden lang geschüttelt. Das auf diese Weise mit KCl imprägnierte Produkt wurde anschließend zunächst 10 Stunden lang bei 150°C und danach noch 18 Stunden bei 450°C getrocknet.

In der gemäß Beispiel 1 beschriebenen Anordnung wurden über diesen Kontakt bei 450°C 40 g/Stunde Tetrachlorkohlenstoff, 4 l/Stunde Wasserstoff und 2 l/Stunde Stickstoff geleitet. Neben 10,9 g/Stunde HCl wurden 23,3 g/Stunde eines Kondensats erhalten mit folgender Produktverteilung:

$CHCl_3$ 2,5 Gew.-%, $CCl_4$ 67,4 Gew.-%, $C_2Cl_4$ 24,6 Gew.-%, $C_2Cl_6$ 1,4 Gew.-%, $C_4Cl_6$ 3,8 Gew.-%.

Umsatz von $CCl_4$ zu $C_2Cl_4$ 26,6 Mol-%, Selektivität 76 Mol-%.

### Beispiel 4

Es wird die Anordnung gemäß Beispiel 1 verwendet. Als Katalysator wird Mordenit in H-Form eingesetzt (Perlen von 2 mm Durchmesser, $SiO_2$-Gehalt 72 Gew.-%, spezifische Oberfläche 470 $m^2$/g).

40 g/Stunde Tetrachlorkohlenstoff und 4 l/Stunde Wasserstoff wurden bei 440°C über den oben charakterisierten Kontakt geleitet. Neben 7,6 g/Stunde Chlorwasserstoff wurden 30,4 g/Stunde Kondensat mit folgender Produktverteilung erhalten:

$CHCl_3$ 1,3 Gew.-%, $CCl_4$ 81,9 Gew.-%, $C_2HCl_3$ 0,1 Gew.-%,
$C_2Cl_4$ 14,8 Gew.-%, $C_2Cl_6$ 0,3 Gew.-%, $C_4Cl_6$ 1,4 Gew.-%.

Der Umsatz von $CCl_4$ zu $C_2Cl_4$ betrug 14,8 Mol-%. Selektivität: 83 Mol-%.

### Beispiel 5

Die Arbeitsweise gemäß Beispiel 4 wurde wiederholt, mit der Abänderung, daß die Reaktionstemperatur 470°C betrug.

Neben 11,6 g/Stunde Chlorwasserstoff wurden 25,6 g/Stunde Kondensat der folgenden Zusammensetzung erhalten:

$CHCl_3$ 1,8 Gew.-%, $CCl_4$ 68,1 Gew.-%, $C_2HCl_3$ 0, 9 Gew.-%,
$C_2Cl_4$ 21,4 Gew.-%, $C_2Cl_6$ 0,2 Gew.-%, $C_4Cl_6$ 2,7 Gew.-%.

Umsatz von $CCl_4$ zu $C_2Cl_4$ : 25,4 Mol-%. Selektivität: 78,2 Mol-%.

### Beispiel 6

Als Kontakt wird ein Mordenit in der Na-Form (3-mm-Kugeln, $SiO_2$-Gehalt 67 Gew.-%, spezifische Oberfläche 440 $m^2$/g) in die gemäß Beispiel 1 beschriebene Anordnung eingesetzt.

Es wurden 40 g/Stunde $CCl_4$, 2 l/Stunde Wasserstoff und 2 l/Stunde Stickstoff über den oben charakterisierten Katalysator geleitet.

Ergebnis: 5,4 g/Stunde HCl, 34 g/Stunde Kondensat der folgenden Zusammensetzung:

$CHCl_3$ 1,6 Gew.-%, $CCl_4$ 86,2 Gew.-%, $C_2Cl_4$ 10,5 Gew.-%, $C_2Cl_6$ 0,03 Gew.-%, $C_4Cl_6$ 1,5 Gew.-%.

Demnach lag der Umsatz von $CCl_4$ zu $C_2Cl_4$ bei 16,5 Mol-% mit einer Selektivität von 77,6 Mol-%.

### Beispiel 7

Es wurde die gemäß Beispiel 4 beschriebene Arbeitsweise wiederholt, mit der Abänderung, daß 10 l/Stunde Wasserstoff als Chlorakzeptor und 5 l/Stunde Kohlendioxid als Trägergas eingesetzt wurden. Die Temperatur der Reaktionszone betrug 390°C.

Es wurden 16,7 g/Stunde Chlorwasserstoff und 15 g Kondensat der folgenden Zusammensetzung gefunden:

$CHCl_3$ 2,4 Gew.-%, $CCl_4$ 56,6 Gew.-%, $C_2HCl_3$ 0,3 Gew.-%,
$C_2Cl_4$ 33,1 Gew.-%, $C_2Cl_6$ 0,02 Gew.-%, $C_4Cl_6$ 3,5 Gew.-%.

Der Umsatz von $CCl_4$ zu $C_2Cl_4$ betrug 23 Mol-%. Die Selektivität in bezug auf die Umwandlung zu $C_2Cl_4$ lag bei 85 Mol-%.

### Beispiel 8

Die Arbeitsweise gemäß Beispiel 7 wurde wiederholt, mit der Abänderung, daß die Temperatur in der Reaktionszone 410°C betrug.

Ergebnis: 21,5 g/Stunde HCl, 11,2 g/Stunde flüssiges Kondensat folgender Zusammensetzung:

$CHCl_3$ 1,6 Gew.-%, $CCl_4$ 24,4 Gew.-%, $C_2HCl_3$ 3,0 Gew.-%,
$C_2Cl_4$ 61,1 Gew.-%, $C_2Cl_6$ 0,2 Gew.-%, $C_4Cl_6$ 8,0 Gew.-%.

Umsatz von $CCl_4$ zu $C_2Cl_4$ 32 Mol-%. Selektivität: 80 Mol-%.

**0 073 055**

### Beispiel 9

318 g Mordenit in der H-Form gemäß Beispiel 4 wurden durch Sprühimprägnierung mit 3 g Rutheniumtrichloridhydrat beladen und bei 150°C 10 Stunden getrocknet. Über den so gewonnenen Kontakt wurde in der Anordnung gemäß Beispiel 1 bei einer Temperatur der Reaktionszone von 390°C 80 g/Stunde $CCl_4$ und 9 l/Stunde Wasserstoff geleitet.

Neben 26 g/Stunde Chlorwasserstoff wurden 54,0 g/Stunde Kondensat mit folgender Produktverteilung gefunden:

$CHCl_3$ 4,8 Gew.-%, $CCl_4$ 67,0 Gew.-%, $C_2HCl_3$ 0,35 Gew.-%,
$C_2Cl_4$ 24,7 Gew.-%, $C_2Cl_6$ 0,1 Gew.-%, $C_4Cl_6$ 2,7 Gew.-%.

Der Umsatz von $CCl_4$ zu $C_2Cl_4$ betrug 31 Mol-% bei einer Selektivität von 77 Mol-%.

### Beispiel 10

342 g Mordenit/H gemäß Beispiel 4 wurden mit einer wäßrigen Lösung von 1,4 g Rutheniumchloridhydrat und 1,5 g Natriumhexachlorrhodenat (III) · 12-Hydrat durch Besprühen imprägniert.

Über den so gewonnenen Kontakt wurden bei einer Temperatur von 450°C in der Reaktionszone 40 g/Stunde $CCl_4$ und 4 l/Stunde Wasserstoff geleitet. Es wurden neben 11,7 g/Stunde Chlorwasserstoff 24 g/Stunde Kondensat der folgenden Zusammensetzung gewonnen:

$CHCl_3$ 3,7 Gew.-%, $CCl_4$ 61,5 Gew.-%, $C_2Cl_4$ 29,0 Gew.-%, $C_2Cl_6$ 0,16 Gew.-%, $C_4Cl_6$ 4,5 Gew.-%.

Umsatz von $CCl_4$ zu $C_2Cl_4$ : 32 Mol-%, Selektivität in bezug auf die Umwandlung von $CCl_4$ zu $C_2Cl_4$ : 79 Mol-%.

### Beispiel 11

383 g Mordenit/Na gemäß Beispiel 6 wurden bei 540°C calciniert und anschließend mit einer Lösung von 650 g Natriumchlorid in 2 l Wasser 5 Stunden bei 70°C behandelt. Schließlich wurde bei 450°C getrocknet.

Über den so gewonnenen Kontakt wurden 40 g/Stunde $CCl_4$, 2 l/Stunde Wasserstoff und 2 l/Stunde Stickstoff geleitet.

Ergebnis: 6,7 g/Stunde HCl und 28,8 g/Stunde Kondensat mit folgender Produktverteilung:

$CHCl_3$ 0,4 Gew.-%, $CCl_4$ 83,1 Gew.-%, $C_2Cl_4$ 11,1 Gew.-%, $C_2Cl_4$ 0,2 Gew.-%, $C_4Cl_6$ 0,65 Gew.-%.

Umsatz von $CCl_4$ zu $C_2Cl_4$ : 15 Mol-%, Selektivität: 90 Mol-%.

### Beispiel 12

Über den Katalysator gemäß Beispiel 12 wurden bei einer Temperatur von 425°C in der Reaktionszone 60 g/Stunde und 10 l/Stunde Wasserstoff geleitet. Es wurden 39,2 g/Stunde Kondensat und 20 g Stunde Chlorwasserstoff ermittelt.

Produktverteilung im Kondensat:

$CHCl_3$ 0,2 Gew.-%, $CCl_4$ 76,7 Gew.-%, $C_2Cl_4$ 19,6 Gew.-%, $C_2Cl_6$ 0,3 Gew.-%, $C_4Cl_6$ 1,4 Gew.-%.

Umsatz von $CCl_4$ zu $C_2Cl_4$ : 23,7 Mol-% mit einer Selektivität von 90,2 Mol-%.

Die gemäß den Beispielen 1 bis 12 beschriebenen Verfahrensweisen wurden jeweils beim Druck der umgebenden Atmosphäre, also bei etwa 1 bar abs. durchgeführt.

### Beispiel 13

Der Katalysator gemäß Beispiel 11 wurde in einen Druckrohrreaktor eingefüllt. 50 g/Stunde Tetrachlormethan und 2,5 l/Stunde Wasserstoff wurden bei einer Reaktionszonentemperatur von 400°C unter einem Druck von 5 bar abs. über den Katalysator geleitet. Es wurden 6,2 g/Stunde Chlorwasserstoff und 40 g/Stunden Kondensat mit folgender Produktverteilung erhalten:

$CHCl_3$ 1,0 Gew.-%, $CCl_4$ 78,3 Gew.-%, $C_2Cl_4$ 14,2 Gew.-%,
$C_2Cl_6$ 0,1 Gew.-%, $C_4Cl_6$ 3,1 Gew.-%, $C_6Cl_6$ 3,3 Gew.-%.

Umsatz von $CCl_4$ zu $C_2Cl_4$ : 21,1 Mol-%, Selektivität 58 Mol-%.

5

**Patentansprüche**

1. Verfahren zur Umwandlung von Tetrachlorkohlenstoff in Perchlorethylen in der Gasphase in Gegenwart von Chlorakzeptoren, dadurch gekennzeichnet, daß die Umwandlung an oberflächenreichen Festkörpern mit einem analytischen SiO$_2$-Gehalt von 60 bis 100 Gew.-% bei Temperaturen von 350 bis 550° C durchgeführt wird.

2. Verfahren nach Anspruch 1, dadurch gekennzeichnet, daß die oberflächenreichen Festkörper eine spezifische Oberfläche von 100 bis 700 m$^2$/g aufweisen.

3. Verfahren nach mindestens einem der Ansprüche 1 bis 2, dadurch gekennzeichnet, daß als Chlorakzeptor Wasserstoff eingesetzt wird.


**Claims**

1. Process for the conversion of carbon tetrachloride into perchloroethylene in the gaseous phase in the presence of chlorine-acceptors, characterized in that the conversion is carried out on solids having a large surface area and an analytical SiO$_2$ content of from 60 to 100% by weight, at temperatures of from 350 to 550° C.

2. Process according to claim 1, characterized in that the large-surface-area solids have a specific surface area of from 100 to 700 m$^2$/g.

3. Process according to at least one of claims 1 and 2, characterised in that hydrogen is used as the chlorine-acceptor.


**Revendications**

1. Procédé pour transformer le tétrachlorure de carbone en perchloréthylène, en phase gazeuse, en présence d'accepteurs de chlore, procédé caractérisé en ce qu'on effectue la transformation au contact de corps solides de grande surface ayant une teneur analytique en SiO$_2$ de 60 à 100% en poids, à des températures de 350 à 550° C.

2. Procédé selon la revendication 1, caractérisé en ce que les corps solides à grande surface ont une surface spécifique de 100 à 700 m$^2$/g.

3. Procédé selon l'une des revendications 1 et 2, caractérisé en ce qu'on utilise l'hydrogène comme accepteur de chlore.